(19) Europäisches Patentamt
European Patent Office
Office européen des brevets

(11) Veröffentlichungsnummer: **0 268 560 A2**

(12) **EUROPÄISCHE PATENTANMELDUNG**

(21) Anmeldenummer: **87810659.0**

(22) Anmeldetag: **13.11.87**

(51) Int. Cl.[4]: **C 07 C 43/174**
A 01 N 31/14

(30) Priorität: **19.11.86 CH 4619/86**
**01.10.87 CH 3822/87**

(43) Veröffentlichungstag der Anmeldung:
**25.05.88 Patentblatt 88/21**

(84) Benannte Vertragsstaaten:
**AT BE CH DE ES FR GB GR IT LI NL**

(71) Anmelder: **CIBA-GEIGY AG**
**Klybeckstrasse 141**
**CH-4002 Basel (CH)**

(72) Erfinder: **Gsell, Laurenz, Dr.**
**Malengasse 56**
**CH-4056 Basel (CH)**

**Ackermann, Peter, Dr.**
**Hangelimattweg 113**
**CH-4148 Pfeffingen (CH)**

(54) **Substituierte Phenoxybenyzl-(dihalogen-dimethyl-cyclopropyl-methyl)-äther.**

(57) Neue substituierte Phenoxybenzyl-dihalogen-dimethyl-cyclopropylmethyl-äther der Formel

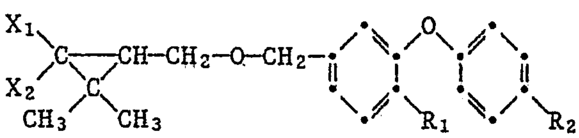

worin
$X_1$ und $X_2$ unabhängig voneinander Fluor, Chlor oder Brom;
$R_1$ Wasserstoff oder Fluor; und
$R_2$ Wasserstoff, Fluor, Chlor, Brom, Methyl, Trifluormethyl oder Methoxy bedeuten, und für den Fall, dass $X_1$ und $X_2$ unterschiedliche Bedeutungen haben, die optischen Isomeren der Verbindung der Formel I; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten, speziell deren larvalen Stadien, sowie von phytopathogenen Pilzen. Die neuen Verbindungen weisen vor allem gute Wirksamkeit gegen pflanzenschädigende Insekten in Reiskulturen auf.

EP 0 268 560 A2

**Beschreibung**

Substituierte Phenoxybenzyl-(dihalogen-dimethyl-cyclopropylmethyl)-äther

Die vorliegende Erfindung betrifft neuartige, substituierte (3-Phenoxybenzyl)-(2,2-dihalogen-3,3-dimethylcyclopropylmethyl)-äther und deren optische Isomere, Verfahren zur Herstellung der neuartigen Verbindungen sowie ihre Verwendung in der Schädlingsbekämpfung.

Die neuen erfindungsgemässen Aether haben die Formel I

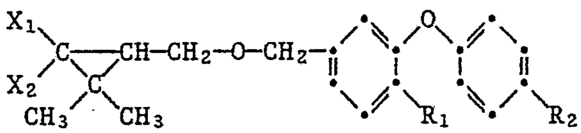
(I)

worin
$X_1$ und $X_2$ unabhängig voneinander Fluor, Chlor oder Brom;
$R_1$ Wasserstoff oder Fluor; und
$R_2$ Wasserstoff, Fluor, Chlor, Brom, Methyl, Trifluormethyl oder Methoxy bedeuten. Die vorliegende Erfindung schliesst auch optische Isomeren von Verbindungen gemäss Formel I ein.

Hervorzuheben wegen ihrer biologischen Wirkung sind Verbindungen der Formel I, worin
$X_1$ und $X_2$ Chlor;
$R_1$ Wasserstoff oder Fluor; und
$R_2$ Wasserstoff, Fluor, Chlor oder Brom
bedeuten.

Die Verbindungen der Formel I werden in an sich bekannter Weise hergestellt, indem man

a) eine Verbindung der Formel II oder IIa

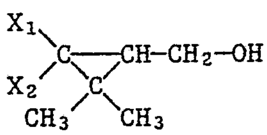
(II)

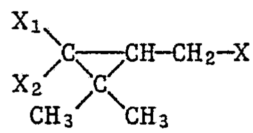
(IIa)

mit einer Verbindung der Formel III bzw. IIIa

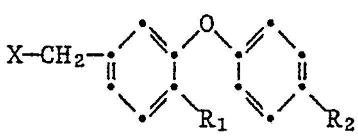
(III)

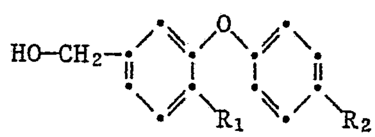
(IIIa)

oder

b) eine Verbindung der Formel IV

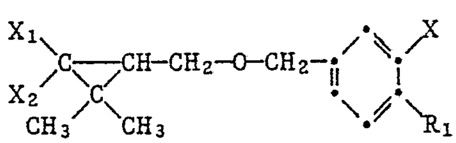
(IV)

mit einer Verbindung der Formel V

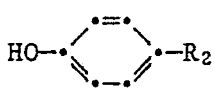
(V)

umsetzt, wobei $X_1$, $X_2$, $R_1$ und $R_2$ die vorstehend angegebenen Bedeutungen besitzen und X für ein Halogenatom, vorzugsweise Brom oder Jod, und im Falle der Verbindungen der Formeln IIa und III auch für die p-Toluolsulfonatgruppe steht.

Die obigen Verfahren werden bei einer Reaktionstemperatur zwischen -10 und 120°C, meist zwischen 20 und 80°C, bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran;

Amide, wie N,N-dialkylierte Carbonsäureamide; aliphatische, aromatische sowie halogenierte Kohlenwasserstoff, insbesondere Benzol, Toluol, Xylole, Chloroform und Chlorbenzol; Nitrile, wie Acetonitril; Dimethylsulfoxid und Ketone wie Aceton und Methylketon sowie Hexan. Die bei Verfahren a) stattfindende Verätherung wird mit vorteil in Gegenwart von Basen, wie Alkalihydroxyden oder -carbonaten, speziell aber Metallhydriden, z.b. Natriumhydrid, durchgeführt. Die bei Verfahren b) stattfindende Verätherung zum Phenyläther wird entweder unter den üblichen Bedingungen der Ullmann-Kondensation bzw. deren Varianten durchgeführt oder entsprechend dem Verfahren a).

Die Ausgangsstoffe der Formeln II, IIa, III, IIIa und V sind bekannt oder können - falls sie neu sind - analog bekannter Methoden hergestellt werden. So wird die Herstellung des substituierten Cyclopropylmethanols der Formel II in "Tetrahedron Letters" 34, 3331-35, beschrieben; die Herstellung dieses Verbindungstyps wird auch in Synthesis 1973, 112, und Helv. Chim. Acta 58, 2595 (1975) erwähnt. Die Phenoxybenzylderivate von Typ der Formeln III und IIIa sowie deren Herstellung sind z.B. bekannt aus der EP-Patentanmeldung 0,125,204, der GB-Patentanmeldung 2,085,006 und der DE-OS 2709355. Die Cyclopropylmethyl-benzyläther der Formel IV sind teilweise neu; sie können hergestellt werden durch Umsetzung des Dihalogendimethylcyclopropylmethanols der Formel II mit einem entsprechend substituierten Benzylderivat der Formel VI

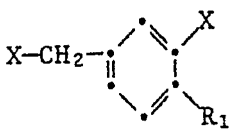

(VI),

worin X und $R_1$ die vorstehend angegebenen Bedeutungen haben.

Aus der EP-Patentschrift Nr. 120,238 sind bereits substituierte Dihalogendimethylcyclopropylmethyl-alkyläther als Mikrobizide bekannt. Diese Verbindungen besitzen jedoch im Gegensatz zu den erfindungsgemässen substituierten Diäthern eine Monoäther-Struktur. Ferner wird in der US-Patentschrift Nr. 4,542,243 ein Verfahren zur Herstellung von 3-Phenoxybenzyl-2-(4-alkoxyphenyl)-2-methylpropyl äthern beschrieben, die sich strukturell von den erfindungsgemässen Verbindungen der Formel I vor allem durch das Fehlen der Cyclopropylmethyl-Gruppierung unterscheiden.

Demgegenüber wurde nun gefunden, dass die neuartigen erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen. In diesem Zusammenhang wird auf die sehr geringe Fisch-Toxizität der erfindungsgemässen Verbindungen hingewiesen, ein für die Anwendung in Reis-Kulturen wichtiger Aspekt.

Insbesondere eignen sich die Verbindungen der Formeln I bzw. Ia) zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Neben ihrer Wirkung gegenüber Mücken und Fliegen, wie z.B. Aëdes aegypti und Musca domestica, können Verbindungen der Formel I auch zur Bekämpfung von pflanzenschädigenden fressenden und saugenden Insekten in Zier- und Nutzpflanzunge, insbesondere in Reiskulturen (z.B. gegen Nilaparuata lugens und Nephotettix cincticeps) sowie in Getreide-, Baumwoll-, Obst- und Gemüsekulturen (z.B. gegen Laspeyresia pomonella, Leptinotarsa decemlineata, Epilachna varivestis, Spodoptera literalis und Heliothis virescens) eingesetzt werden. Die Verbindungen der Formel I zeichnen sich auch durch gute Wirkung gegen larvale Insektenstadien und Nymphen, insbesondere von fressenden Schadinsekten, aus. Auch können die Verbindungen der Formel I mit ausgezeichnetem Erfolg gegen pflanzenschädigende Zikaden, speziell in Reiskulturen, eingesetzt werden.

Die Verbindungen eignen sich ferner zur Bekämpfung von Ektoparasiten, z.B. Lucilia sericata, sowie von Zecken an Haus- und Nutztieren, z.B. durch Tier-, Stall- und Weidebehandlung.

Es wurde weiterhin gefunden, dass Verbindungen der Formel I ein für praktische Bedürfnisse sehr günstiges biozides Spektrum gegen phytopathogene Mikroorganismen, insbesondere gegen Fungi aufweisen. Sie besitzen sehr vorteilhafte kurative, systemische und insbesondere präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben. Als Fungizide sind die Wirkstoffe der Formel I z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); insbesondere wirken sie gegen die Klasse der Ascomyceten (z.B. Venturia, Podosphaera, Erysiphe, Monilinia, Uncinula). Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten,

insbesondere pflanzenschützende Mittel, sowie deren Verwendung auf dem Agrarsektor oder verwandten Gebieten.

Als Zielkulturen für den fungiziden, pflanzenschützenden Einsatz gelten im Rahmen dieser Erfindung beispielsweise folgende Pflanzenarten: Getriede (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen (Compositen). Die Verbindungen der Formel I sind als Fungizide insbesondere in Reiskulturen wirksam, speziell gegen Piricularia oryzae.

Die Wirkung der erfindungsgemässen Verbindungen bzw. der sie enthaltenden Mittel lässt sich durch Zusatz von anderen Pestiziden wesentlich verbreitern und an gegebene Umstände anpassen. Als insektizide und/oder akarizide Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit andere Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werde. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthenol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Dieser Salze liegen vorzugsweise als Halogenide, Methylsulfate oder Aethylsulfate vor. z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten - auf das Gewicht bezogen -in der Regel 0,1 bis 99 %, insbesondere 0,1 bis 95 %, eines Wirkstoffes der Formel I oder Kombinationen davon mit anderen Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung des (3-Phenoxy-4-fluorbenzyl)-(2,2-dichlor-3,3-dimethylcyclopropylmethyl)-äthers

Es werden in einer Stickstoffatmosphäre 2,2 g 2,2-Dichlor-3,3-dimethylcyclopropylmethanol und 3,2 g 3-Phenoxy-4-fluorbenzylbromid, gelöst in 20 ml eines Gemisches aus Toluol und Dimethylformamid (1/1) unter Eiskühlung (0-5°C) zu 0,6 g Natriumhydrid (50%ige Dispersion in Mineralöl) in 50 ml Toluol/Dimethylformamid (1/1) tropfenweise hinzugefügt. Nach Abklingen der Reaktion wird der Ansatz während 16 Stunden bei Raumtemperatur ausgerührt auf gesättigte Ammoniumchlorid-Lösung gegossen und mit Toluol extrahiert. Die vereinigten Toluol-Extrakte werden mit gesättigter Kochsalzlösung gewaschen, über $MgSO_4$ getrocknet und am Rotationsverdampfer eingeengt. Das erhaltene Rohprodukt wird an Kieselgel mit Hexan/Toluol (1:1) chromatographiert. Die Titelverbindung der Formel

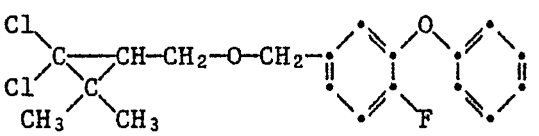

wird als klares Oel vom Brechungsindex $n_D^{24}$ = 1,5488 (Verbindung Nr. 1) erhalten.

Wie vorstehend beschrieben, werden auch die folgenden Verbindungen der Formel I erhalten:

| Verbindung Nr. | $X_1$ | $X_2$ | $R_1$ | $R_2$ | physikal. Daten |
|---|---|---|---|---|---|
| 2 | Cl | Cl | H | Br | $n_D^{22}$ = 1,5759 |
| 3 | Cl | Cl | H | Cl | $n_D^{22}$ = 1,5680 |
| 4 | Cl | Cl | H | H | $n_D^{24}$ = 1,5524 |
| 5 | Cl | Cl | H | F | $n_D^{25}$ = 1,5400 |
| 6 | F | F | H | F | $n_D^{21}$ = 1,5178 |
| 7 | Br | Cl | H | H | $n_D^{23}$ = 1,5572 |
| 8 | Br | Br | H | H | $n_D^{22}$ = 1,5725 |
| 9 | Br | Br | H | F | $n_D^{22}$ = 1,5662 |
| 10 | Br | Cl | H | F | $n_D^{22}$ = 1,5438 |
| 11 | Br | Cl | F | H | $n_D^{22}$ = 1,5452 |
| 12 | Fl | Cl | F | H | $n_D^{22}$ = 1,5275 |
| 13 | F | Cl | H | F | $n_D^{22}$ = 1,5219 |
| 14 | Br | Br | F | H | $n_D^{22}$ = 1,5608 |
| 15 | F | Cl | H | H | $n_D^{22}$ = 1,5360 |
| 16 | F | F | F | H | $n_D^{22}$ = 1,5200 |
| 17 | F | F | H | H | $n_D^{21}$ = 1,5208 |
| 18 | Cl | Cl | F | Cl | $n_D^{22}$ = 1,5381 |
| 19 | Cl | Cl | F | $CH_3$ | $n_D^{24}$ = 1,5365 |

Tabelle: (Fortsetzung)

| Verbindung Nr. | $X_1$ | $X_2$ | $R_1$ | $R_2$ | physikal. Daten |
|---|---|---|---|---|---|
| 20 | Cl | Cl | F | F | $n_D^{24} = 1,5362$ |
| 21 | F | Cl | H | $-OCH_3$ | $n_D^{25} = 1,5444$ |
| 22 | Br | Br | H | $-OCH_3$ | $n_D^{25} = 1,5759$ |
| 23 | F | F | F | F | $n_D^{25} = 1,5082$ |
| 24 | Cl | Cl | F | $-CH_3$ | $n_D^{24} = 1,5365$ |
| 25 | Cl | Cl | H | $-OCH_3$ | $n_D^{25} = 1,5498$ |

Die folgenden Verbindungen der Formel I sind ebenfalls wie in Beispiel I beschrieben erhältlich:

| $X_1$ | $X_2$ | $R_1$ | $R_2$ |
|---|---|---|---|
| F | Cl | H | $CF_3$ |
| F | Br | H | Cl |

Beispiel 2:

Formulierungen für flüssige Wirkstoffe der Formel I gemäss Beispiel 1 oder Kombinationen dieser Wirkstoffe mit anderen Insektiziden oder Akariziden (% = Gewichtsprozent):

| 2.1. Emulsions-Konzentrate | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 2.3. Granulate | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss Beispiel 1 oder Kombinationen dieser Wirkstoffe mit anderen Insektiziden oder Akariziden (% = Gewichtsprozent):

2.5. Spritzpulver

| | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.
Man erhält Spritzpulver, die sich mit Wasser zu Suspensionen jeder gewünschten Konzentration verdünnen lassen.

2.6. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

2.8. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

2.9. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

2.10. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Lucilia sericata (Larven)

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 4: Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Die Verbindungen Nr. 1, 2, 4 und 5 gemäss Beispiel 1 zeigen 80-100 % Wirkung in diesem Test.

Beispiel 5 Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22°C und etwa 60 % relativer Luftfeuchtigkeit durchgeführt.

Verbindungen gemäss Beispiel 1 zeigen gute Wirkung in diesem Test.

Beispiel 6: Kontakt-Wirkung auf Laodelphax striatellus und Nilaparvata lugens (Nymphen);

Der Test wird an wachsenden Pflanzen durchgeführt. Dazu werden jeweils 8 Reispflanzen (Dicke des Stengels 4 mm) mit einer Höhe von ca. 20 cm in Töpfe (Durchmesser von 8 cm) eingepflanzt.

Die Pflanzen werden auf einem Drehteller mit 100 ml einer wässrigen Zubereitung enthaltend 400 ppm des jeweiligen formulierten Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des Adultstadiums über 6 Tage an der behandelten Pflanze gehalten. Die Auswertung auf %-Mortalität erfolgt 6 Tage nach der Behandlung.

Verbindung Nr. 1 gemäss Beispiel 1 zeigt 80-100 % Wirkung in diesem Test gegen Nilaparvata lugens.

Beispiel 7: Systemische Wirkung auf Nilaparvata lugens:

Etwa 10 Tage alte Reispflanzen (ca. 10 cm hoch) werden in einen Plastik-Becher eingestellt, der 150 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren. Dann wird die Reispflanze mit 20 Nymphen von Nilaparvata lugens im N2 bis N3 Stadium besiedelt und mit einem Plastikzylinder abgedeckt. Der Versuch wird bei 26°C und 60 % relativer Luftfeuchtigkeit mit einer Beleuchtungesperiode von 16 Stunden durchgeführt. Nach 6 Tagen wird auf die Anzahl der abgetöteten Testtiere, im Vergleich zu unbehandelten Kontrollen, bonitiert. Damit wird festgestellt, ob der über die Wurzel aufgenommene Wirkstoff die Testtiere an den oberen Pflanzenteilen abtötet.

Verbindungen Nr. 1 gemäss Beispiel 1 zeigt im obigen Versuch 80-100 % Wirkung (Mortalität) gegen Nilaparvata lugens.

Beispiel 8: Insektizide Frassgift-Wirkung

Ca. 25 cm hohe eingetopfte Baumwollplanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis- bzw. Heliothis virescens-larven im ersten larvalen Stadium besiedelt. Der Versuch wird bei 24°C und etwa 60 % relativer Luftfeuchtigkeit durchgeführt. Nach 120 Stunden wird die %-Mortalität der Test-Insekten gegenüber unbehandelten Kontrollen bestimmt.

Die Verbindung Nr. 1 gemäss Beispiel 1 zeigt 80-100%ige Wirkung gegen Spodoptera-Larven in diesem Test.

Beispiel 9: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer wässrigen Wirkstoffemulsion enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze gehalten. Der Versuch wird bei einer Temperatur von ca. 26°C, bei 55 % relativer Luftfeuchtigkeit und mit einer Belichtungsperiode von 16 Stunden durchgeführt.

Die erfindungsgemässen Verbindungen gemäss Beispiel 1 zeigen in diesem Test gute Wirkung.

Beispiel 10: Wirkung gegen Bodeninsekten (Diabrotica balteata)

Es werden 5 Maiseimlinge von 1-3 cm Länge sowie eine Filterpapier-Rondelle in eine wässrige Wirkstofflösung enthaltend etwa 4 vol.-% Aceton eingetaucht. Der Wirkstoffgehalt der verwendeten Lösung beträgt 400 ppm. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der Ansatz wird bei ca. 24°C und 40-60 % relativer Luftfeuchtigkeit und Tageslicht gehalten. Die Bonitur erfolgt nach 6 Tagen gegenüber unbehandelten Kontrollansätzen.

Die erfindungsgemässen Verbindungen Nr. 1 und 4 zeigen in diesem Test eine Wirkung von 80-100 % (Mortatität).

Beispiel 11: Wirkung gegen tierparasitäre Milben

Von mit Dermanyssus gallinae befallenen Hühnern werden Ansätze bestehend aus etwa 50 Milben verschiedener Stadien (Mischpopulation: Larven, Nymphen und Adulte) entnommen. Die Milbenansätze werden jeweils mit einer wässrigen Emulsion, Suspension bzw. Lösung enthaltend 400 ppm des zu prüfenden Wirkstoffes benetzt. Hierzu werden die Milbenansätze in einem Reagenzglas mit der den Wirkstoff enthaltenden flüssigen Zubereitung übergossen; die Flüssigkeit wird anschliessend mit Hilfe eines Wattebausches aufgesogen. Die so benetzten und behandelten Milben verbleiben während 72 Stunden in dem Reagenzglas. Nach dieser Zeit wird die Abtötung der behandelten Milben gegenüber unbehandelten Kontrollansätzen ermittelt.

Verbindungen Nr. 1 gemäss dem vorstehenden Beispiel 1 zeigt 100%-ige Wirkung im obigen Test.

Beispiel 12: Wirkung gegen Zecken: Abtötungswirkung in verschiedenen Entwicklungsstadien

Als Testobjekte pro Ansatz werden nicht vollgesogene Larven (jeweils ca. 50), Nymphen (jeweils 5) oder Adulte (jeweils 5) der Zeckenarten Rhipicephalus bursa, Amblyomma hebraeum und Boophilus microplus verwendet. Die Testtiere (in der angegebenen Anzahl) werden für kurze Zeit in 2 bis 3 ml einer wässrigen Emulsion der zu untersuchenden Verbindung mit einer Konzentration von 400 ppm getaucht, die sich in einem Reagenzglas befindet. Die Reagenzgläser werden dann mit einem Wattepfropfen verschlossen und 10 Minuten nach dem Eintauchen der Testtiere geschüttelt. Dabei wird die Wirkstoff-Emulsion von den Wattepfropfen aufgesogen und die benetzten Testtiere in den so kontaminierten Reagenzgläsern belassen. Die Auswertung (% - Mortalität) erfolgt für Larven nach 3 Tagen und für Nymphen und Adulte nach 14 Tagen.

Verbindung Nr. 1 gemäss dem vorstehenden Beispiel 1 zeigt im obigen Test 100%-ige Wirkung (Mortatlität) gegen Nymphen und Adulte von Amblyomma hebraeum.

Beispiel 13: Wirkung gegen Piricularia oryzae auf Reispflanzen-Residual-protektive Wirkung

a) Reispflanzen werden nach 2-wöchiger Anzucht mit einer aus Spritzpulver des Wirkstoffes hergestellten Spitzbrühe (enthaltend 200 ppm Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall ausgewertet.

b) Systemische Wirkung:

Zu 2-wöchigen, in Töpfen befindlichen Reispflanzen wird eine aus Spritzpulver des Wirkstoffes hergestellte Spritzbrühe gegossen (0,006 % bezogen auf das Erdvolumen). Daraufhin werden die Töpfen mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95-100 % relativer Luftfeuchigkeit und ca. 24°C wird der Pilzbefall ausgewertet.

Reispflanzen, die mit einer Spritzbrühe behandelt werden, die als Aktivsubstanz eine Verbindung der Formel I gemäss Beispiel 1 enthält, zeigen im Vergleich zu unbehandelten Kontrollpflanzen (100 % Befall) nur geringen Pilzbefall. So reduziert z.B. im obigen Test a) die Verbindung Nr. 1 den Pilzbefall auf etwa 20 %.

**Patentansprüche**

1. Verbindung der Formel I

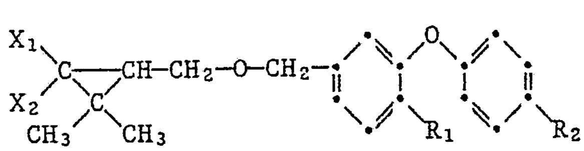

(I)

worin

$X_1$ und $X_2$ unabhängig voneinander Fluor, Chlor oder Brom;

$R_1$ Wasserstoff oder Fluor; und

$R_2$ Wasserstoff, Fluor, Chlor, Brom, Methyl, Trifluormethyl oder Methoxy bedeuten, und für den Fall, dass $X_1$ und $X_2$ unterschiedliche Bedeutungen haben, die optischen Isomeren der Verbindung der Formel I.

2. Verbindung gemäss Anspruch 1 der Formel I, dadurch gekennzeichnet, dass

$X_1$ und $X_2$ Chlor;

$R_1$ Wasserstoff oder Fluor; und

$R_2$ Wasserstoff, Fluor, Chlor oder Brom

bedeuten.

3. Verbindung gemäss Anspruch 2 der Formel

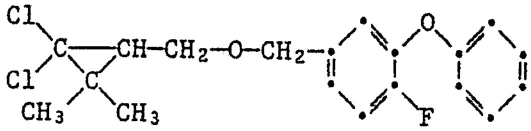

4. Verbindung gemäss Anspruch 2 der Formel

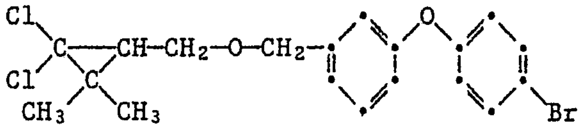

5. Verbindung gemäss Anspruch 1 der Formel

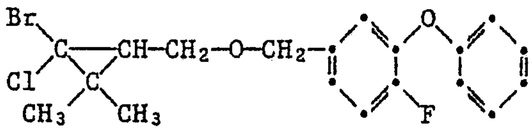

6. Verbindung gemäss Anspruch 2 der Formel

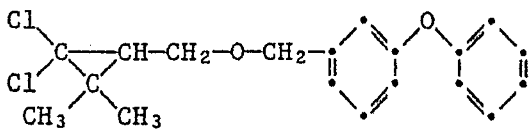

7. Verbindung gemäss Anspruch 1 der Formel

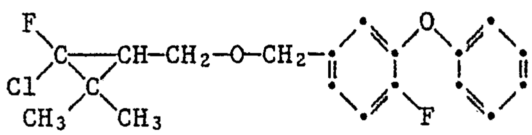

8. Verfahren zur Herstellung einer Verbindung der Formel I gemäss Anspruch 1, dadurch gekennzeichnet, dass man
a) eine Verbindung der Formel II oder IIa

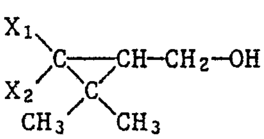 (II)

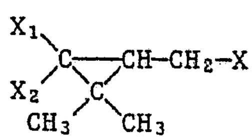 (IIa)

mit einer Verbindung der Formel III bzw. IIIa

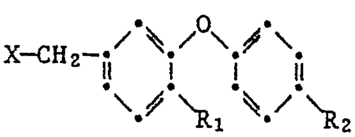 (III)

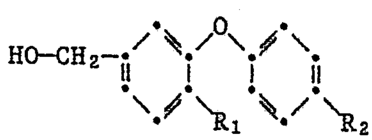 (IIIa)

oder

b) eine Verbindung der Formel IV

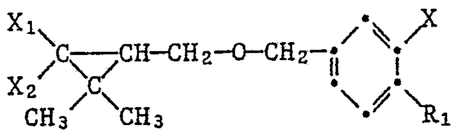

(IV)

mit einer Verbindung der Formel V

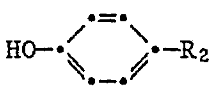

(V)

umsetzt, wobei $X_1$, $X_2$, $R_1$ und $R_2$ die in Anspruch 1 angegebenen Bedeutungen besitzen und X für ein Halogenatom, vorzugsweise Brom oder Jod, und im Falle der Verbindungen der Formeln IIa und III auch für die p-Toluolsulfonatgruppe steht.

9. Schädlingsbekämpfungsmittel, welches als aktive Komponente eine Verbindung gemäss einem der Ansprüche 1 bis 7 zusammen mit geeigneten Trägern und/oder anderen Zuschlagstoffen enthält.

10. Verwendung einer Verbindung gemäss einem der Ansprüche 1 bis 7 zur Bekämpfung von Insekten und Vertretern der Ordnung Akarina an Tieren und Pflanzen.

11. Verwendung gemäss Anspruch 10 zur Bekämpfung von pflanzenschädigenden Insekten.

12. Verwendung gemäss Anspruch 11 zur Bekämpfung von pflanzenschädigenden Insekten in Reiskulturen.

13. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 7 oder mit einem Mittel enthaltend neben Zusatz- und Trägerstoffen eine pestizid wirksame Menge dieser Verbindung, in Kontakt bringt oder behandelt.

14. Verwendung einer Verbindung gemäss einem der Ansprüche 1 bis 7 zur Bekämpfung von pflanzenschädigenden Pilzen.

15. Verwendung gemäss Anspruch 14 zur Bekämpfung von pflanzenschädigenden Pilzen in Reiskulturen.

16. Verfahren zur Bekämpfung von pflanzenschädigenden Pilzen, dadurch gekennzeichnet, dass man die Pilze bzw. deren Sporen oder den von ihnen befallenen Ort mit einer fungizid wirksamen Menge einer Verbindung der Formel I gemäss einem der Ansprüche 1 bis 7 oder mit einem Mittel enthaltend neben Zusatz- und Trägerstoffen eine fungizid wirksame Menge dieser Verbindung, in Kontakt bringt oder behandelt.